(19) Europäisches Patentamt
European Patent Office
Office européen des brevets

(11) Veröffentlichungsnummer: **0 355 031**
**A2**

(12) # EUROPÄISCHE PATENTANMELDUNG

(21) Anmeldenummer: **89115174.8**

(22) Anmeldetag: **17.08.89**

(51) Int. Cl.4: **C07H 19/073 , C07D 405/04 , A61K 31/70 , A61K 31/505**

(30) Priorität: **17.08.88 DD 319023**

(43) Veröffentlichungstag der Anmeldung:
**21.02.90 Patentblatt 90/08**

(84) Benannte Vertragsstaaten:
**AT BE CH DE ES FR GB GR IT LI NL SE**

(71) Anmelder: **Akademie der Wissenschaften der DDR**
**Otto-Nuschke-Strasse 22/23**
**DDR-1086 Berlin(DD)**

(72) Erfinder: **Matthes, Eckert, Dr.-med.**
**Karower Chaussee 129**
**DDR-1115 Berlin(DD)**
Erfinder: **von Janta-Lipinski, Martin, Dr.**
**Mittelweg 75**
**DDR-1185 Berlin(DD)**
Erfinder: **Scholz, Dieter, Dr.**
**Heinrich-Roller-Strasse 16**
**DDR-1055 Berlin(DD)**
Erfinder: **Gaertner, Klaus**
**Karower Chaussee 157**
**DDR-1115 Berlin(DD)**
Erfinder: **Schildt, Jürgen**
**Winsstrasse 58**
**DDR-1055 Berlin(DD)**
Erfinder: **Lehmann, Christine**
**Walter-Friedrich-Strasse 5**
**DDR-1115 Berlin(DD)**
Erfinder: **Langen, Peter, Prof.Dr.**
**Karower Chaussee 219**
**DDR-1115 Berlin(DD)**
Erfinder: **Rosenthal, Hans-Alfred, Prof.Dr.**
**Märkisches Ufer 14**
**DDR-1020 Berlin(DD)**

(74) Vertreter: **Patentanwälte Beetz·sen. - Beetz jun. Timpe - Siegfried - Schmitt-Fumian-Mayr**
**Steinsdorfstrasse 10**
**D-8000 München 22(DE)**

(54) **Substituierte Pyrimidinnucleoside, Verfahren zu ihrer Herstellung und sie enthaltende pharmazeutische Mittel.**

(57) Es werden neue, gegen durch Viren bzw. Retroviren verursachte Infektionen wirksame, substituierte Pyrimidinnucleoside der Formel I, II und III

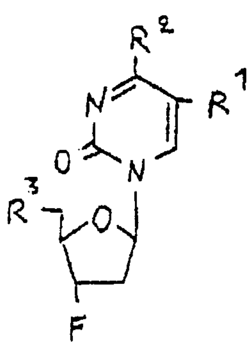
I

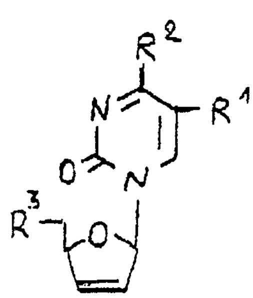
II

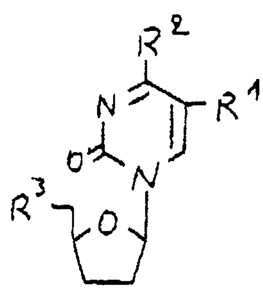
III

EP 0 355 031 A2

in der bedeuten:
$R^1$ Wasserstoff, Halogen, Azido, aliphatisches Alkyl mit 1 bis 5 Kohlenstoffatomen, vorzugsweise mit 1 bis 3 Kohlenstoffatomen, oder Alkenyl mit 2 bis 5 Kohlenstoffatomen,
$R^2$ Wasserstoff, Thio, Thiomethyl, Hydroxylamino oder Alkylamino,
$R^3$ Hydroxyl, O-Acetyl, O-Palmitoyl, O-Alkoxycarbonyl, Mono-, Di-, oder Triphosphorsäure, -alkaliphosphat, -ammoniumphosphat oder -alkylammoniumphosphat oder eine Vorstufe für die Hydroxylgruppe,
und sie enthaltende pharmazeutische Mittel beschrieben.
Ein besonders geeigneter Wirkstoff ist 1-(2,3-Didesoxy-3-fluor-ß-D-ribofuranosyl)-4-thiothymin.

## Substituierte Pyrimidinnucleoside, Verfahren zu ihrer Herstellung und sie enthaltende pharmazeutische Mittel

Die Erfindung betrifft substituierte Pyrimidinnucleoside, die gegen durch Viren bzw. Retroviren verursachte Infektionen bei Mensch und Tier, insbesondere HIV-Infektionen (AIDS) beim Menschen wirksam sind, ihre Herstellung und ihre Verwendung in Form entsprechender pharmazeutischer Mittel.

AIDS ist eine seit wenigen Jahren bekannte, durch das HIV (HTLV III/LAV, Human T-Lymphotropie Virus Type III/Lymphadenopathy-Associated Virus) verursachte und zum Tode führende Infektionskrankheit. Als ursächlich muß die Zerstörung der T-Helfer-Zellen durch das AIDS-Virus angesehen werden. Als Folge der daraus resultierenden Abwehrschwäche treten schwere opportunistische Infektionen, das Kaposi-Sarkom und eine sog. AIDS-Enzephalopathie auf. Eine wirksame und verträgliche antivirale Therapie fehlt bisher. Angriffspunkt dafür kann u.a. die Virus-kodierte Reserve Transcriptase sein, ein Enzym, dessen Hemmung die weitere intrazelluläre Virusvermehrung verhindern und damit seine Ausbreitung im Körper unterbinden könnte. Die ersten klinisch erprobten Hemmstoffe der HIV-Revertase, wie z.B. Suramin (Germanin$^{R}$) und HPF 23 haben die notwendige Verträglichkeit und die erhoffte Wirksamkeit noch nicht erreicht, lediglich das 3′-Azidothymidin ($N_3TdR$) (DE-A-35 00 606) besitzt bei AIDS-Patienten mit einer Pneumocystis-carinii-Pneumonie eindeutig lebensverlängernde Wirkungen, die von Verbesserungen klinischer und neurologischer Befunde sowie der zeitweisen Wiederherstellung bestimmter immunologischer Funktionen begleitet sind (Fischl et al., The New England J. of Medicine 317, 185 (1987)). Demgegenüber machen toxische Nebenwirkungen des 3′-Azidothymidins auf das Knochenmark bei etwa 50 % der behandelten Patienten Bluttransfusionen erforderlich, was besonders auf die Notwendigkeit nach selektiveren Hemmstoffen der HIV-Reverse Transcriptase hinweist, während auch deren Wirksamkeit zu erhöhen ist.

Der Erfindung liegt die Aufgabe zugrunde, neuartige Wirkstoffe und entsprechende pharmazeutische Mittel und deren Herstellung und Verwendung anzugeben, die zur prophylaktischen sowie therapeutischen Behandlung von AIDS bzw. von durch Viren bzw. Retroviren verursachten Infektionen verwendbar sind und gegenüber den bekannten Mitteln geringere Toxizität bzw. Nebenwirkungen aufweisen.

Die Aufgabe wird gemäß den Ansprüchen 1, 14 und 15 gelöst.

Vorteilhafte Weiterbildungen sind Gegenstand der abhängigen Ansprüche 2 bis 13.

Die erfindungsgemäßen Pyrimidinnucleoside besitzen die allgemeine Formel I, II oder III

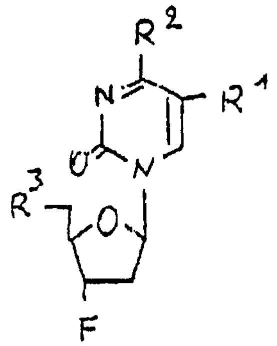

I

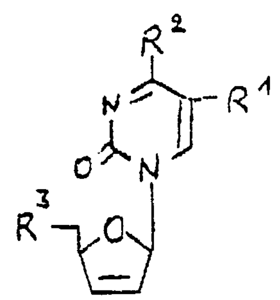

II

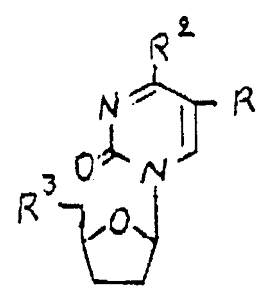

III

in der bedeuten:

$R^1$ Wasserstoff, Halogen, Azido, aliphatisches Alkyl mit 1 bis 5 Kohlenstoffatomen, vorzugsweise mit 1 bis 3 Kohlenstoffatomen, oder Alkenyl mit 2 bis 5 Kohlenstoffatomen,

$R^2$ Wasserstoff, Thio, Thiomethyl, Hydroxylamino oder Alkylamino,

$R^3$ Hydroxyl, O-Acetyl, O-Palmitoyl, O-Alkoxycarbonyl, Mono-, Di-, oder Triphosphorsäure, -alkaliphosphat, -ammoniumphosphat oder -alkylammoniumphosphat oder eine Vorstufe für die Hydroxylgruppe.

Sie stellen wirksame Mittel gegen durch Viren bzw. Retroviren verursachte Infektionen dar.

Die erfindungsgemäßen Verfahren zur Herstellung der Pyrimidinnucleoside der Formel I, II und III sind dadurch gekennzeichnet, daß zur Herstellung der

- 4-Thioverbindungen die entsprechenden (1H, 3H)-Pyrimidin-2,4-dion-Verbindungen mit Phosphorpentasulfid in Pyridin erhitzt werden (J.J. Fox et al, J. Amer. Chem. Soc. (1959), 81, 178-187);
- 4-Hydroxylamino- bzw. 4-Alkylaminoverbindungen die entsprechenden Thioverbindungen mit dem entsprechenden Alkylamin bzw. Hydroxylamin umgesetzt werden (J.J. Fox et al, J. Amer. Chem. Soc. (1959) 81, 178-187);
- (1H)-Pyrimidin-2-on-Derivate die entsprechenden 2,4-Dionverbindungen mit Hydrazin zu 4-Hydrazino-Derivaten umgesetzt und mit Silberoxid oxydiert werden;
- 2′,3′-Didexosy-2′,3′-didehydro-Verbindungen aus den entsprechenden 3′,5′-Di-O-mesylverbindungen die Mesylgruppen abgespalten werden (J.P. Horwitz et al, J. Org. Chem. (1966), 31, 205-211);
- 2′,3′-Didesoxynucleoside die entsprechenden ungesättigten Verbindungen mit 10 proz. Palladium/Kohle als Katalysator hydriert werden (J.P. Horwitz et al, J. Org. Chem. (1966), 31, 205-211).

Erfindungsgemäße Verbindungen der Formel I, II und III sind:
1-(2,3-Didesoxy-3-fluor-ß-D-ribofuranosyl)-5-methyl-(1H)-pyrimidin-2-on
1-(2,3-Didesoxy-3-fluor-ß-D-ribofuranosyl)-4-thiothymin
1-(2,3-Didesoxy-3-fluor-ß-D-ribofuranosyl)-4-methylmercapto-5-methyl-(1H)-pyrimidin-2-on
1-(2,3-Didesoxy-3-fluor-ß-D-ribofuranosyl)-4-hydroxylamino-5-methyl-(1H)-pyrimidin-2-on
5-Chlor-1-(2,3-didesoxy-3-fluor-ß-D-ribofuranosyl)-4-thiouracil
1-(2,3-Didesoxy-3-fluor-ß-D-ribofuranosyl)-(1H)-pyrimidin-2-on
1-(2,3-Didesoxy-3-fluor-ß-D-ribofuranosyl)-4-thiouracil
1-(2,3-Didesoxy-3-fluor-ß-D-ribofuranosyl)-4-hydroxylamino-(1H)-pyrimidin-2-on
1-(2,3-Didesoxy-ß-D-glycero-pent-2-enofuranosyl)-4-thiothymin
1-(2,3-Didesoxy-ß-D-glycero-pent-2-enofuranosyl)-5-methyl-(1H)-pyrimidin-2-on
1-(2,3-Didesoxy-ß-D-glycero-pent-2-enofuranosyl)-4-hydroxylamino-(1H)-pyrimidin-2-on
1-(2,3-Didesoxy-ß-D-glycero-pent-2-enofuranosyl)-4-dimethylamino-(1H)-pyrimidin-2-on.

Die erfindungsgemäßen pharmazeutischen Mittel enthalten mindestens eine Verbindung der allgemeinen Formel I und/oder II und/oder III als Wirkstoff zusammen mit mindestens einem verträglichen Träger und ggfs. mit anderen therapeutischen Mitteln. Die Mittel werden als Einheitsdosis hergestellt oder so, daß ein Mehrfaches des Wirkstoffs enthalten ist. Jeder Träger muß verträglich sein, mit den anderen Bestandteilen der Mittel vereinbar und darf für den Patienten nicht schädlich sein.

Die Mittel schließend solche ein, die für orale, rektale, nasale, topische, vaginale oder parenterale (einschließlich subkutane, intramuskuläre, intravenöse und intradermale) Verabreichung geeignet sind. Ein bzw. mehrere Wirkstoff(e) wird (werden) mit dem Träger, der sich aus einem oder mehreren Begleitbestandteilen zusammensetzt, in Kontakt gebracht und, falls erforderlich, in eine entsprechende Form gebracht.

Die Mittel für eine orale Verabreichung werden in Form von Dragees, Tabletten, Kapseln, als Pulver oder Granulat so hergestellt, daß sie jeweils eine bestimmte Menge des aktiven Bestandteils enthalten. Ebenso können sie als Lösung oder als Suspension hergestellt werden. Gegebenenfalls werden Geschmacksmittel oder andere übliche Mittel zugesetzt. Mittel für eine rektale Verabreichung werden als Zäpfchen mit einer geeigneten Base hergestellt. Mittel für eine vaginale Verabreichung werden als Pessare, Tampons, Cremes, Gele, Pasten, Schäume oder Spray-Produkte hergestellt.

Die Mittel für die parenterale Verabreichung können als Einheitsdosis des Wirkstoffs oder als Mehrfachdosis vorgesehen werden. Dafür können sie in Ampullen, Fiolen oder in einem gefriergetrockneten Zustand gelagert werden. Unmittelbar zubereitete Injektionslösungen und Suspensionen können aus sterilen Pulvern, Granulat und Tabletten erhalten werden. Das erfolgt z.B. durch Lösen der Substanz in physiologischer Kochsalzlösung, Glukose oder anderen für die i.v. Injektion bzw. Infusion geeigneten Medien.

Folgende Verbindungen erwiesen sich als besonders wirksam:
1-(2,3-Didesoxy-3-fluor-ß-D-ribofuranosyl)-5-methyl-(1H)-pyrimidin-2-on (I);
3′-Desoxy-3′-fluor-4-thiothymidin (I);
1-(2,3-Didesoxy-3-fluor-ß-D-ribofuranosyl)-4-methylmercapto-5-methyl-(1H)-pyrimidin-2-on (I);
5-Chlor-1-(2,3-didesoxy-3-fluor-ß-D-ribofuranosyl)-4-thiouracil (I);
1-(2,3-Didesoxy-3-fluor-ß-D-ribofuranosyl)-(1H)-pyrimidin-2-on (I);
1-(2,3-Didesoxy-3-fluor-ß-D-ribofuranosyl)-4-thiouracil (I);
1-(2,3-Didesoxy-ß-D-glycero-pent-2-enofuranosyl)-4-dimethylamino-(1H)-pyrimidin-2-on (II);
1-(2,3-Didesoxy-ß-D-glycero-pent-2-enofuranosyl)-4- hydroxylamino-(1H)-pyrimidin-2-on (II);
3′-Desoxy-4-thiothymidin (III).

Die Wirksamkeit einiger der erfindungsgemäßen Verbindungen wird wie folgt belegt:

1. <u>Selektive Hemmung der HIV-assoziierten Reverse Transcriptase (HIV-RT) durch 3′-Fluor-4-thiothymidin-</u>

triphosphat

Für diese Untersuchungen wurde die Wirkung der Verbindung auf die Aktivität sowohl der HIV-RT als auch der zellulären DNA-Polymerase entsprechend den von Matthes et al., in Biochem. Biophys. Res. Commun. 148, 78 (1987) beschriebenen Methoden bestimmt. Den Enzymansätzen wurde 3′-Fluor-4-thiothymidintriphosphat in verschiedenen Konzentrationen zugesetzt, aus den erhaltenen Hemmkurven wurden die Konzentrationen von 3′-Fluor-4-thiothymidintriphosphat bestimmt, die die untersuchten Polymerasen zu jeweils 50 % hemmen ($ID_{50}$). Für die HIV-RT wurde eine $ID_{50}$ von 0,3 µmol/l, für die DNA-Polymerase, dem für die zelluläre Replikation hauptverantwortlichen Enzym, eine $ID_{50}$ von 140 µmol/l und für die DNA-Polymerase ß, dem zellulären DNA-Reparatur-Enzym, eine $ID_{50}$ von 7 µmol/l bestimmt. Damit hemmt 3′-Fluor-4-thiothymidintriphosphat die HIV-RT 700-mal stärker als die zelluläre DNA-Polymerase und besitzt damit eine weitgehend selektive Wirkung auf die HIV-Replikation.

2. Hemmung der zytopathischen Wirkung von HIV auf T-Lymphozyten

Eine Infektion von T-Lymphozyten mit HIV führt unter Invitro-Bedingungen innerhalb weniger Tage zum Zelltod. Die von Harada et al., Science 229, 563 (1985) beschriebenen MT-4- Zellen wurden in einem Testsystem (Matthes et al., Biochem. Biophys., Res. Commun. 153, 825 (1988) verwendet, um zu prüfen, inwieweit die erfindungsgemäßen Nucleoside die zelltötende Wirkung von HIV-1 (HTLV IIIa) auf diese T-Zellinie aufheben können. In der Tabelle sind die Konzentrationen wiedergegeben, die zu einem 50-%igen Schutz der MT-4-Zellen führen. Außerdem ist die antiproliferative Wirksamkeit der untersuchten Nucleoside aufgeführt und zwar als die Konzentration, die die Zellvermehrung der MT-4-Zellen um 50 % herabsetzt ($CD_{50}$).

3. Zytotoxizität von 3′-Fluor-4-thiothymidin gegenüber Zellkulturen in vitro

Die Ergebnisse an den MT-4-Zellen haben gezeigt, daß 3′-Fluor-4-thiothymidin die Zellvermehrung dieser T-Zelllinie kaum beeinflußt. Eine Reihe weiterer menschlicher Zellinien wurde in die Untersuchung der antiproliferativen Wirkung von 3′-Fluor-4-thiothymidin einbezogen und folgende $CD_{50}$-Werte gefunden:

1. REH, akute lymphatische Leukämie = 500 µmol/l
2. HELF1, menschliche embryonale Lungenfibroblasten = 700 µmol/l
3. H-8, immortalisierte T-Zellinie = > 800 µmol/l
4 Molt-4, immportalisierte T-Zellinie = >1000 µmol/l.

Darüberhinaus besitzt 3′-Fluor-4-thiothymidin in Konzentrationen bis 100 µmol/l gegenüber den koloniebildenden Zellen des Knochenmarks der Maus (GM-CFU) keinerlei wachstumsbeeinträchtigende Wirkung.

Zusammenfassend kann 3′-Fluor-4-thiothymidin daher als wirksamer Hemmstoff der HIV-Replikation mit sehr geringer Zytotoxizität charakterisiert werden.

Tabelle

| Vergleich der antiviralenund antiproliferativenWirksamkeitvon Nucleosidanaloga an MT-4-Zellen | | |
|---|---|---|
| Untersuchtes Nucleosid | 50 % antivirale Dosis ($ED_{50}$; µmol/l) | 50 % zytotoxische Dosis ($CD_{50}$; µmol/l) |
| 1-(2,3-Didesoxy-3-fluor-β-D-ribofuranosyl)-4-thiothymin | 1,8 | 480 |
| 1-(2,3-Didesoxy-3-fluor-β-D-ribofuranosyl)-4-thiouracil | 15 | 130 |
| 1-(2,3-Didesoxy-3-fluor-β-D-ribofuranosyl)-4-methylmercapto-5-methylpyrimidin-2-on | >1000 | >1000 |
| 1-(2,3-Didesoxy-3-fluor-β-D-ribofuranosyl)-4-methylmercapto-pyrimidin-2-on | 12 | 50 |
| 1-(2,3-Didesoxy-3-fluor-β-D-ribofuranosyl)-4-hydroxylamino-5-methylpyrimidin-2-on | 110 | 300 |
| 1-(2,3-Didesoxy-3-fluor-β-D-ribofuranosyl)-4-hydroxylamino-pyrimidin-2-on | 100 | 100 |

Die folgenden Beispiele erläutern die Herstellung der erfindungsgemäßen Verbindungen.

**Beispiel 1**

**1-(2,3-Didesoxy-3-fluor-ß-D-ribofuranosyl)-4-thiouracil**

1,3 mmol 1-(2,3-Didesoxy-3-fluor-ß-D-ribofuranosyl)-uracil werden in 45 ml Pyridin gelöst und mit 1,1 ml Acetanhydrid versetzt. Das Reaktionsgemisch wird über Nacht bei Raumtemperatur stehengelassen. Die Lösung wird eingeengt, der Rückstand in Toluol gelöst und erneut zur Trockne konzentriert. Der Vorgang wird mehrfach wiederholt. Der schließlich gewonnene Rückstand wird in 80 ml Pyridin gelöst, mit 3,9 mmol Phosphorpentasulfid versetzt und 6 h unter Rückfluß erhitzt. Nach dieser Reaktionszeit werden weitere 1,3 mmol Phosphorpentasulfid zugegeben (J. J. Fox et al., J. Am. Chem. Soc., 1959, 81, 178; G. Kowollik et al. J. Prakt. Chem., 1973, 315, 895) und es wird 6 h unter Rückfluß erhitzt. Nach dem Erkalten des Reaktionsgemisches wird der flüssige Anteil abgetrennt und im Vakuum zur Trockne eingeengt. Der resultierende Rückstand wird an Kieselgel 40 (MERCK) mit Chloroform als Eluiermittel säulenchromatographisch gereinigt. Aus den entsprechenden Fraktionen wird 1-(5-O-Acetyl-2,3-didesoxy-3-fluor-ß-D-ribofuranosyl)-4-thiouracil isoliert und ergibt nach dem umkristallisieren aus 90 % Ethanol 97 mg,
Fp. 140-140,5 °C. 15 mg (0,06 mmol) dieser Verbindung werden in 2 ml Methanol gelöst und mit 2 ml bei 0 °C mit Ammoniak gesättigtem Methanol versetzt. Nach 24 h wird die Lösung zur Trockne eingeengt. Die Verbindung wird als Glas erhalten.
MS: m/z 246 ($C_9H_{11}N_2O_3FS$, $M^+$), 128 ($C_4H_4N_2OS$, Base + H), 119 ($C_5H_8O_2F$), Zuckerrest).

**Beispiel 2**

**1-(5-O-Acetyl-2,3-didesoxy-3-fluor-ß-D-ribofuranosyl)-4-thiothymin**

400 mg (1,4 mmol) 5′-O-Acetyl-3′-fluorthymidin werden mit 933 mg Phosphorpentasulfid in 32 ml Pyridin 24 h unter Rückfluß erhitzt. Nach dem Vertreiben des Lösungsmittels wird der Rückstand an Kieselgel 40 säulenchromatographisch getrennt und liefert die gewünschte Verbindung als gelbes Öl.
MS: m/z 302 ($C_{12}H_{15}N_2O_4SF$, $M^+$), 161 ($C_7H_{10}O_3F$, Zuckerrest), 142 ($C_5H_8N_2OS$, Base).

**Beispiel 3**

**1-(2,3-Didesoxy-3-fluor-ß-D-ribofuranosyl)-4-thiothymin**

18 mg (0,07 mmol) der im Beispiel 2 erhaltenen Verbindung werden in 2 ml bei 0 °C mit Ammoniak gesättigtem Methanol gelöst und 24 h bei Raumtemperatur stehengelassen. Nach dem Entfernen des Lösungsmittels wird 1-(2,3-Didesoxy-3-fluor-ß-D-ribofuranosyl)-4-thiothymin als glasiger Rückstand isoliert.
MS: m/z 260 ($C_{10}H_{13}N_2O_3FS$, $M^+$), 142 ($C_5H_6N_2OS$, Base + H), 119 ($C_5H_8O_2F$, Zuckerrest).

**Beispiel 4**

**1-(5-O-Acetyl-2,3-didesoxy-3-fluor-ß-D-ribofuranosyl)-4-hydroxylamino-(1H)-pyrimidin-2-on**

70 mg (0,24 mmol) 1-(5-O-Acetyl-2,3-didesoxy-3-fluor-ß-D-ribofuranosyl)-4-thiouracil werden in 5 ml Methanol gelöst, mit 1 ml Hydroxylamin in 12 ml Methanol versetzt und 4 h unter Rückfluß erhitzt. Die dünnschichtchromatographische Kontrolle der Reaktionslösung zeigt eine vollständige Reaktion des Ausgangsproduktes. Das Lösungsmittel wird im Vakuum entfernt, der Rückstand wird an Kieselgel 40 mit Chloroform (2 % Methanol) als Eluiermittel gereinigt. Aus den entsprechenden Fraktionen werden 60 mg

der gewünschten Verbindung erhalten, die aus 90 % Ethanol Kristalle ergeben. Fp. 192-193 °C.
MS: m/z 287 ($C_{11}H_{14}H_3O_5F$, $M^+$), 161 ($C_7H_{10}O_3F$, Zuckerrest), 127 ($C_4H_5N_2O_3$, Base + H).

## Beispiel 5

### 1-(2,3-Didesoxy-3-fluor-β-D-ribofuranosyl)-4-hydroxylamino-(1H)-pyrimidin-2-on

50 mg der in Beispiel 4 erhaltenen Verbindung werden in üblicher Weise mit Ammoniak/Methanol entacetyliert. Die Verbindung wird als glasartiger Rückstand isoliert.
MS: m/z 245 ($C_9H_{12}N_3O_4F$, $M^+$), 127 ($C_4H_5N_2O_3$, Base + H), 119 ($C_6H_8O_2F$, Zuckerrest).

## Beispiel 6

### 1-(2,3-Didesoxy-3-fluor-β-D-ribofuranosyl)-4-methylmercapto-5-methyl-(1H)-pyrimidin-2-on

Eine Lösung von 70 mg (0,23 mmol) 1-(5-O-Acetyl-2,3-didesoxy-3-fluor-β-D-ribofuranosyl)-4-thiothymin, 0,23 ml 1 n Natronlauge und 0,044 ml Methyljodid in 4 ml 50-%igem Methanol wird 2 h bei Raumtemperatur stehengelassen und dann mit Essigsäure auf einen pH-Wert von 5 gebracht. Nach dem Vertreiben des Lösungsmittels werden aus dem Rückstand 45 mg 1-(2,3-Didesoxy-3-fluor-β-D-ribofuranosyl)-4-methylmercapto-5-methyl-(1H)-pyrimidin-2-on gewonnen, die nach dem Umkristallisieren aus Ethanol 32 mg des reinen Produkts ergeben. Fp. 192-194 °C.
MS: m/z 274 ($C_{11}H_{15}N_2O_3FS$, $M^+$), 156 ($C_8H_8N_2OS$, Base + H), 119 ($C_5H_8O_2F$, Zuckerrest).

## Beispiel 7

### 1-(5-O-Acetyl-2,3-didesoxy-3-fluor-β-D-ribofuranosyl)-4-hydroxylamino-5-methyl-(1H)-pyrimidin-2-on

93 mg (0,31 mmol) 1-(5-O-Acetyl-2,3-didesoxy-3-fluor-β-D-ribofuranosyl)-4-thiothymin werden mit einer Lösung von 1 ml Hydroxylamin in 15 ml Methanol versehen und 4 h unter Rückfluß erhitzt. Der nach dem Entfernen des Lösungsmittels im Vakuum verbleibende Rückstand wird an 75 g Kieselgel 40 mit Chloroform als Eluiermittel säulenchromatographisch gereinigt. Aus den entsprechenden Fraktionen werden insgesamt 51 mg eines Produktes erhalten, das nach dem Umkristallisieren aus Methanol 35 mg 1-(5-O-Acetyl-2,3-didesoxy-3-fluor-β-D-ribofuranosyl)-4-hydroxylamino- 5-methyl-(1H)-pyrimidin-2-on ergibt. Fp. 180-181 °C.
MS: m/z 301 ($C_{12}H_{16}N_3O_5F$, $M^+$), 161 ($C_7H_{10}O_3F$, Zuckerrest), 141 ($C_5H_7N_3O_2$, Base + H).

## Beispiel 8

### 1-(2,3-Didesoxy-3-fluor-β-D-ribofuranosyl)-4-hydroxylamino-5-methyl-(1H)-pyrimidin-2-on

45 mg der im Beispiel 7 hergestellten Verbindung werden in herkömmlicher Weise mit Ammoniak/Methanol behandelt und ergeben nach dem Kristallisieren 27 mg 1-(2,3-Didesoxy-3-fluor-β-D-ribofuranosyl)-4-hydroxylamino-5-methyl-(1H)-pyrimidin-2-on.
MS: m/z 259 ($C_{10}H_{14}N_3O_4F$, $M^+$), 141 ($C_5H_7N_3O_2$, Base + H), 119 ($C_5H_8O_2F$, Zuckerrest).

## Beispiel 9

### 1-(5-O-Acetyl-2,3-didesoxy-β-D-glycero-pent-2-enofuranosyl)-4-thiothymin

Zu einer Lösung von 450 mg (2 mmol) 3′-Desoxy-2′-Thymidinen in 10 ml Pyridin wird 1 ml Acetanhydrid gegeben. Das Reaktionsgemisch wird 10 h bei Raumtemperatur stehengelassen. Das Lösungsmittel wird im Vakuum vertrieben. Der verbleibende Rückstand wird in absolutem Ethanol gelöst. Die Lösung wird zur Trockne eingeengt. Dieser Vorgang des Lösens in Ethanol und Vertreibens des Lösungsmittels wird solange wiederholt, bis ein kristallines Material erhalten wird, das aus Methanol umkristallisiert wird. Fp. 183 °C.
MS: m/z 266 ($C_{12}H_{14}N_2O_6$, $M^+$), 141 ($C_7H_9O_3$, Zuckerrest), 126 ($C_5H_6N_2O_2$, Base + H).

Das im Vakuum getrocknete 1-(5-O-Acetyl-2,3-didesoxy-ß-D-glycero-pent-2-enofuranosyl)-thymin wird in 25 ml absolutem Pyridin gelöst und mit 2 g Phosphorpentasulfid 48 h unter Rückfluß erhitzt. Nach dem Erkalten wird vom Unlöslichen dekantiert und die Lösung im Vakuum zur Trockne eingeengt. Der resultierende Rückstand wird an Kieselgel 40 mit Chloroform als Eluiermittel säulenchromatographisch getrennt. Aus den das Produkt enthaltenden Fraktionen werden nach dem Umkristallisieren aus Methanol 265 mg 1-(5-O-Acetyl-2,3-didesoxy-ß-D-glycero-pent-2-enofuranosyl)-4-thiothymin gewonnen.
MS: m/z 282 ($C_{12}H_{14}N_2O_4S$, $M^+$), 141 ($C_7H_9O_3$, Zuckerrest), 142 ($C_5H_6N_2OS$, Base + H).

## Beispiel 10

### 1-(2,3-Didesoxy-ß-D-glycero-pent-2-enofuranosyl)-4-thiothymin

Zu 100 mg der im Beispiel 9 synthetisierten Verbindung werden 10 ml Methanol, das bei 0 °C mit Ammoniak gesättigt wurde, zugegeben. Das Reaktionsgemisch wird 12 h bei Raumtemperatur stehengelassen und dann zur Trockne eingeengt. Aus Methanol/Ether werden Kristalle erhalten.
Fp. 117-118 °C.
MS: m/z 240 ($C_{10}H_{12}N_2O_3S$, $M^+$), 209 ($C_9H_9N_2O_2S$, M-$H_2O$), 142 ($C_5H_8N_2OS$, Base + H).

## Beispiel 11

### 1-(2,3-Didesoxy-3-fluor-ß-D-ribofuranosyl)-4-thiothymin-5′-triphosphat

60 mg 1-(2,3-Didesoxy-3-fluor-ß-D-ribofuranosyl)-4-thiothymin (0,21 mmol) werden in 0,5 ml Trimethylphosphat gelöst und bei 0 °C mit 0,0423 ml Phosphoroxychlorid versetzt (Yoshikawa et al., Tetrahedron Lett., 5065 (1967)). Das Reaktionsgemisch wird 2 h bei 0 °C stehengelassen, dann mit 10 ml Eiswasser behandelt und mit Triethylamin neutralisiert. Nach dem Hinzufügen von 300 ml Wasser wird die Reaktionslösung an DEAE-Sephadex säulenchromatographisch mit 0-0.3 mol/l Triethylammoniumhydrogencarbonat als Eluiermittel getrennt. Aus den entsprechenden Fraktionen wird das 5′-Monophosphat isoliert. Dieses Produkt wird mittels DOWEX WX 8 (Pyridinium-Form) in das Tri-n-butyl- ammoniumsalz übergeführt, nach der Entfernung des Lösungsmittels mit N,N′-Carbonyldiimidazol aktiviert und mit Tetra-n-tributylammoniumpyrophosphat in DMF in bekannter Weise (D.E. Hoard et al., J. Am. Chem. Soc. 87, 1785 (1965)), in das 5′-Triphosphat umgewandelt. Die Verbindung wird an DEAE-Sephadex mit einem Gradienten von 0-0,5 mol/l Triethylammoniumhydrogencarbonat eluiert. Aus den entsprechenden Fraktionen wird ein Produkt erhalten, aus dem nach dem Entfernen des Eluiermittels mit Natriumjodid in bekannter Weise das Natriumsalz der Titelverbindung gewonnen wird.
Phosphatbestimmung: 16 %.

## Ansprüche

1. Substituierte Pyrimidinnucleoside der allgemeinen Formel I, II und III

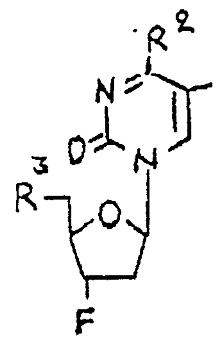 I 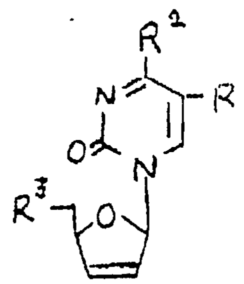 II 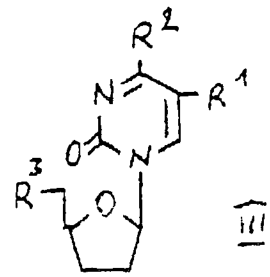 III

in der bedeuten:

R' Wasserstoff, Halogen, Azido, aliphatisches Alkyl mit 1 bis 5 Kohlenstoffatomen, vorzugsweise mit 1 bis 3 Kohlenstoffatomen oder Alkenyl mit 2 bis 5 Kohlenstoffatomen,

$R^2$ Wasserstoff, Thio, Thiomethyl, Hydroxylamino oder Alkylamino

$R^3$ Hydroxyl, O-Acetyl, O-Palmitoyl, O-Alkoxycarbonyl, Mono-, Di-, oder Triphosphorsäure, -alkaliphosphat, -ammoniumphosphat oder -alkylammoniumphosphat oder eine Vorstufe für die Hydroxylgruppe.

2. 1-(2,3-Didesoxy-3-fluor-ß-D-ribofuranosyl)-5-methyl-(1H)-pyrimidin-2-on.

3. 1-(2,3-Didesoxy-3-fluor-ß-D-ribofuranosyl)-4-thiothymin.

4. 1-(2,3-Didesoxy-3-fluor-ß-D-ribofuranosyl)-4-methylmercapto-5-methyl-(1H)-pyrimidin-2-on.

5. 1-(2,3-Didesoxy-3-fluor-ß-D-ribofuranosyl)-4-hydroxylamino-5-methyl-(1H)-pyrimidin-2-on.

6. 5-Chlor-1-(2,3-didesoxy-3-fluor-ß-D-ribofuranosyl)-4-thiouracil.

7. 1-(2,3-Didesoxy-3-fluor-ß-D-ribofuranosyl)-(1H)-pyrimidin-2-on.

8. 1-(2,3-Didesoxy-3-fluor-ß-D-ribofuranosyl)-4-thiouracil.

9. 1-(2,3-Didesoxy-3-fluor-ß-D-ribofuranosyl)-4-hydroxylamino-(1H)-pyrimidin-2-on.

10. 1-(2,3-Didesoxy-ß-D-glycero-pent-2-enofuranosyl)-4-thiothymin.

11. 1-(2,3-Didesoxy-ß-D-glycero-pent-2-enofuranosyl)-5- methyl-(1H)-pyrimidin-2-on.

12. 1-(2,3-Didesoxy-ß-D-glycero-pent-2-enofuranosyl)-4-hydroxylamino-(1H)-pyrimidin-2-on.

13. 1-(2,3-Didesoxy-ß-D-glycero-pent-2-enofuranosyl)-4-dimethylamino-(1H)-pyrimidin-2-on.

14. Verfahren zur Herstellung der substituierten Pyrimidinnucleoside nach einem der Ansprüche 1 bis 13, dadurch gekennzeichnet, daß

- zur Herstellung der 4-Thioverbindungen die entsprechenden (1H, 3H)-Pyrimidin-2,4-dion-Verbindungen mit Phosphorpentasulfid in Pyridin erhitzt werden,
- zur Herstellung der 4-Hydroxylamino- oder 4-Alkylaminoverbindungen die entsprechenden Thioverbindungen mit dem entsprechenden Alkylamin oder Hydroxylamin umgesetzt werden,
- zur Herstellung der (1H)-Pyrimidin-2-on-Derivate die entsprechenden 2,4-Dionverbindungen mit Hydrazin und Silberoxid umgesetzt werden,
- zur Herstellung der 2′,3′-Didesoxy-2′,3′-didehydro-Verbindungen aus den entsprechenden 3′,5′-Di-O-mesylverbindungen die Mesylgruppen abgespalten werden,
- zur Herstellung der 2′,3′-Didesoxynucleoside die entsprechenden ungesättigten Verbindungen mit 10 proz. Palladium/Kohle als Katalysator hydriert werden.

15. Pharmazeutische Mittel, insbesondere zur Prophylaxe und/oder Behandlung von durch Viren, insbesondere Retroviren verursachten Infektionen, gekennzeichnet durch mindestens ein substituiertes Pyrimidinnucleosid der allgemeinen Formel I und/oder II und/oder III nach einem der Ansprüche 1 bis 13 als Wirkstoff in üblichen galenischen Hilfs-, Träger- und/oder Verdünnungsmitteln.